# EUROPÄISCHE PATENTSCHRIFT

(11) **EP 1 942 916 B1**
(45) Veröffentlichungstag und Bekanntmachung des Hinweises auf die Patenterteilung: **19.12.2012**
(21) Anmeldenummer: 06804825.5
(22) Anmeldetag: 23.10.2006
(51) Int. Cl.: A61K 36/82, A61P 17/00, A61K 36/00, A61K 31/047, A61K 45/06, A61K 8/97, A61Q 17/04, A61Q 19/00

(54) **Mittel zum Schutz von Hautzellen**
Agent for protecting skin cells
Moyen de protection des cellules dermatites

(30) Priorität: 02.11.2005 CH 17552005
(43) Veröffentlichungstag der Anmeldung: 16.07.2008
(73) Patentinhaber: Omnimedica AG, 8952 Schlieren (CH)
(72) Erfinder: HOLZGANG, Hans-Emanuel, CH-8049 Zürich (CH)
(74) Vertreter: Becker Kurig Straus
(86) Internationale Anmeldenummer: PCT/CH2006/000591
(87) Internationale Veröffentlichungsnummer: WO 2007/051326

(56) Entgegenhaltungen:
- WO-A-01/00038
- WO-A-2005/004827
- US-B1- 6 399 046
- LEE SOOCHUN ET AL: "Effects of chronic polyphenol or vitamin ingestion on antioxidative activity during exercise in rats." März 2003 (2003-03), FASEB JOURNAL, VOL. 17, NR. 4-5, PAGE(S) ABSTRACT NO. 199.3 URL - HTTP://WWW.FASEBJ.ORG/ , FASEB MEETING ON EXPERIMENTAL BIOLOGY: TRANSLATING THE GENOME; SAN DIEGO, CA, USA; APRIL 11-15, 2003 , XP009076727 ISSN: 0892-6638 das ganze Dokument
- UHLENBRUCK G ET AL: "EIGENSCHAFTEN VON GRUENEM TEE: EINE UEBERSICHT" DEUTSCHE ZEITSCHRIFT FUER ONKOLOGIE, HEIDELBERG, DE, Bd. 30, Nr. 3, 1998, Seiten 57-63, XP000881942 ISSN: 0931-0037

## Beschreibung

Die Erfindung betrifft ein Mittel zum Schutz von Hautzellen.

Die DE-U-29604205 offenbart ein Hautschutzspray mit hohem Wassergehalt und Pflanzenextrakten wie Meristemextrakt oder Alpha-Bisabolol, die als Radikalfänger dienen sowie Vitamin E oder Vitaminderivate. Der hohe Wasseranteil stammt sowohl von den Pflanzenextrakten als auch zugegebenem Wasser. Die hochwasserhaltige Lösung soll kühlen und einen klebrig-fettigen Eindruck auf der Haut vermeiden.

Ein Hautpflegemittel gemäss DE-U-20204160 enthält mindestens einen Wirkstoff einer Alge, Wirkstoffe von Bupleurum Chinensis (Wurzel) sowie Vitamine A, C und/oder E. Es soll eine prophylaktische Wirkung bei Belastung durch Radikale und eine restrukturierende Wirkung bei Lichtalterung entfalten. Ebenso sollen dermatologische Befunde wie Juckreiz oder Schuppung vermindert werden.

Ein weiteres Hautpflegemittel auf natürlicher Basis ist in der DE-A-4318280 beschrieben, welches Extrakte von Ringelblumenblüten, Kamilleblüten und ggf. weiteren Pflanzen wie zum Beispiel Ulmenrinde oder Leinsamen enthält. Die Zusammensetzung soll der kosmetischen Behandlung von Hautunregelmässigkeiten, eingeschlossen Schuppenflechte oder Neurodermitis dienen.

Der Erfindung liegt die Aufgabe zugrunde, ein weiteres Mittel zum Schutz von Hautzellen zu entwickeln, das sowohl eine präventive als auch eine leistungssteigernde Wirkung entfalten kann. Die Aufgabe ist mit den Merkmalen des Patentanspruchs 1 gelöst.

Das Mittel bzw die Zusammensetzung enthält aus Grüntee extrahierte Polyphenole, die schnell in die Haut einziehen und ebenfalls gut resorbiert werden können. Diese sind so extrahiert und stabilisiert, dass sie bereits in sehr geringer Dosis appliziert eine schützende Wirkung zeigen.

Bevorzugt enthält die Kombination auch die Vitamine A, C und/oder E und/oder pflanzliche Isoflavone, zum Beispiel Rotklee um eine synergistische Wirkung zu erreichen.

Anwendungsmöglichkeiten ergeben sich in Kombination mit:
➢ UV-Filtem für Sonnenschutzmittel, wodurch die Effektivität des Schutzes bei UV-bedingten Hautschäden erhöht wird. Es ist eine Kombination mit chemischen oder physikalischen Filtern (z.B. Titandioxid, Zinkoxid) möglich, die Konzentration des Mittels in diesen sollte mindestens 0,4% im Endprodukt betragen (soll der Sonnenfilter z. B. 5% einer Sonnenschutzlotion o. dgl. ausmachen, müssten ca. 5-10% des Filters das erfindungsgemässe Mittel enthalten). Einzelne Filter können auch untereinander und mit dem Mittel kombiniert werden;
**➢** weiteren Substanzen, wie zum Beispiel Vitaminen, Coenzymen, Vitaminderivaten, Spurenelementen, Enzymen, Aminosäuren oder Substanzen, die die körpereigene Produktion von solchen stimulieren.

Weitere Einsatzmöglichkeiten bestehen als:
➢ Kortinsonersatz (Ersatz von Kortisonen oder kortisonähnlichen synthetischen Substanzen zur Anwendung auf der Haut oder durch Inhalation und Anwendung auch gegen entzündliche Erkrankungen;
➢ Applikation zur geistigen und körperlichen Leistungssteigerung, im Zusammenhang mit Alzheimertherapien, Chelatbildnern oder in Kombination mit DMSA u. a. (nicht erfindungsgemäß)
➢ Einsatz in Hautschutzprodukten, Hautschutz bei Bestrahlungstherapien, Personalschutz, Schutz vor Zellschäden bei radioaktiver oder elektromagnetischer Strahlung;
➢ Einsatz bei Ulzeras, Diabetespatienten oder dort, wo Corticoide zur Anwendung gelangen.

Das Mittel ist demzufolge sehr gut als Haut schutz und als Radikalfänger (nicht erfindungsgemäß) auch in Nahrungsmitteln (ebenso zur Leistungssteigerung) einsetzbar, d.h. zur äusserlichen, wie (nicht erfindungsgemäß) zur innerlichen, therapeutischen wie medizinischen Anwendung geeignet.

Denkbar ist weiterhin ein Ersatz von Tanninen zur Linderung von Juckreiz bei Infektionen (z. B. Windpocken oder Röteln), wo auch andere Stoffe wie z. B. Zinkoxid angewandt werden.

Möglich erscheint aufgrund der antiviralen Aktivität ebenfalls die Anwendung bei grippalen Infekten (Influenza, HIV, Herpes, Vogelgrippe o. a.), auch in Kombinationen, die die antivirale Aktivität unterstützen (nichterfendungsgemäß). Weitere Anwendungen bestehen in der Krebstherapie in Kombination mit Taxol, AC-, CMF- oder EC-Therapien oder. Misteltherapie.

Um die Wirkung des Mittels zu verbessern, können Derivate von diesem gebildet werden, eingeschlossen der Schutz funktioneller Gruppen oder einzelner Moleküle, deren Substitution oder die Hinzufügung anderer Verbindungen. Umgekehrt können auch einzelne Bestandteile synthetisiert oder zu einem neuen Molekül kondensiert werden um diese wieder mit dem Mittel zu kombinieren und dessen Gesamtwirkung zu optimieren.

Um mögliche Nebenwirkungen zu vermeiden, ist das Mittel frei von organischen Lösungsmitteln und enthält vorzugsweise nur Wasser als Lösungsmittel.

Vorteilhaft kann das Mittel für die topische Verwendung mikroverkapselt oder nanoverkapselt sein.

Verfahren zur Herstellung des Mittels:

Das Extrahieren erfolgt mittels Wasser bei Temperaturen im Bereich von 50°C bis 120°C und bei Drücken im Bereich von 1 bar bis 5 bar. Eine kostengünstige Variante arbeitet bei Umgebungsdruck, ca. 1 bar absolut, und verwendet Temperaturen im Bereich von 50°C bis 98°C. Bei Bedarf kann auch mit Überdruck, zwischen 1 bar absolut und 5 bar absolut, gearbeitet werden, wobei Temperaturen zwischen 50°C und 120°C verwendet werden.

Das Filtrieren erfolgt mittels eines Filterbetts, wobei als Filterbett ein Oxidbett-Filter, ein Polymerbett-Filter oder Mischformen dieser beiden Filtertypen verwendet werden. Besonders bevorzugt sind poröse oder partikuläre Kieselgel-Filter, Glasfaser-Filter, Cellulose-Filter oder anorganisch-organische Mischformen wie Kieselgel mit aufgepfropften organischen Gruppen wie z.B. Alkylgruppen.

Bei einer besonders bevorzugten Ausführung enthält das Filterbett mindestens eine stationäre Filterbettphase aus nicht-polarem Material, insbesondere ein poröses Kieselgel-Gerüst der Kieselgel-Partikel, deren Oberflächen mit nicht-polaren organischen Gruppen bestückt sind (sog. Revers-Phasen-Kieselgele, reversed phase silica gel). Dadurch werden beim Filtrieren die lipophilen Anteile des Extraktes entfernt, so dass ein Grüntee-Extrakt erhalten wird, das frei von organischen Lösungsmitteln ist.

Die Porengrössen bzw. Korngrössen oder Faserdurchmesser des verwendeten Filterbetts liegen zweckmässigerweise im Bereich von 0,01 mm bis 0,5 mm und vorzugsweise im Bereich von 0,05 mm bis 0,2 mm. Besonders bevorzugt sind auch polydisperse Filterbett-Schüttungen, insbesondere mit nicht-polaren Oberflächen.

Über die Einstellung und Auswahl der oben genannten Filterparametër, wie z.B. Korngrössen, Porengrössen, polares oder nicht-polares Filtermaterial, kann das Spektrum der Aminosäuren-Anteile des erfindungsgemässen Mittels eingestellt werden.

Beim anschliessenden Konzentrieren des Extraktes erfolgt eine Aufkonzentration der von Wasser verschiedenen Inhaltsstoffe auf 40 Gew.% bis 100 Gew.%. Durch moderates Abdampfen wird das Extrakt zu einem dünnflüssigen bis dickflüssigen Sirup mit 40 Gew.% bis 80 Gew.% Inhaltsstoffe (von Wasser verschiedene Stoffe) eingedickt. Alternativ kann eine Sprühtrocknung oder Walzentrocknung des Extraktes durchgeführt werden, wobei auf 90 Gew.% bis 100 Gew.% Inhaltsstoffe im oben genannten Sinne angereichert wird. Die oben genannten Gewichtsprozente (Gew.%) verstehen sich als Masse der Summe aller von Wasser verschiedenen Inhaltsstoffe bezogen auf die Gesamtmasse.

Es hat sich auch überraschenderweise gezeigt, dass das erfindungsgemässe Herstellungsverfahren zu einem Mittel führt, dessen Glutamat-Anteil bzw. Glutaminsäure-Anteil am gesamten Aminosäure-Anteil grösser als 60%, insbesondere zwischen 60% und 90% ist.

Bei einer besonders wirksamen Ausführung ("Aminosäure-Spektrum") des erfindungsgernässen Mittels liegen die relativen Anteile bestimmter Aminosäuren (d.h. die Gewichtsanteile der jeweiligen Aminosäure bezogen auf die Gesamtmasse aller in dem Mittel enthaltenen Aminosäuren) in den folgenden Bereichen:

| | |
|---|---|
| Glutaminsäure: | 60% - 80% |
| Asparaginsäure: | 10% - 14% |

und insbesondere:

| | |
|---|---|
| Serin: | 2% - 4% |
| Glycin: | 2% - 4% |
| Alanin: | 2% - 3% |
| Valin: | 1% - 3% |
| Threonin: | 1% - 3% |

Das Mittel kann sowohl dermatologisch verwendet werden als auch (nicht erfindungsgemäß) eingenommen werden. Die dermatologische Verwendung kann kosmetischer (Anti Ageing) oder medizinischer Natur (Hemmung von Entzündungen, Krebsprävention, Beeinflussung des Immunsystems auf der Haut oder Beeinflussung zelleigener Reparaturmechanismen) sein. Die Einnahme des Mittels kann ebenfalls medizinischer Natur mit den vörgenannten Anwendungsbereichen, zuzüglich Allergiereduktion sein. Weiterhin kann das Mittel (nicht erfindngsgemäß) zur Nahrungsergänzung eingenommen werden zwecks Leistungssteigerung, Anti Ageing oder allgemein zum Gesundheitsschutz.

Die Applikation des Mittels ist mit den üblichen Darreichungsformen möglich, zum Beispiel als Creme oder Spray.

Ein weiterer Verwendungsaspekt des Mittels ist sein Einsatz als Genmodulator für Enzyme, zum Beispiel werden Apoptosepromotoren auf Genebene beeinflusst (z. B. Regulierung von Matrix Metallo Proteinasen MMP1 oder MMP2).

Die Erfindung wird nachfolgend in einem Ausführungsbeispiel näher beschrieben. Eine weisse, stabilisierte Hautcreme enthält Grüntee-Extrakte mit stabilisierten reaktiven Gruppen (Polyphenole) und die Vitamine A, C und E zum regelmässigen Auftrag auf Hautpartien, zum Beispiel mit Altersflecken oder Melanome. Die Wirkstoffe befinden sich nur in wässriger Lösung.

Bereits nach kurzzeitiger Anwendung zeigt sich eine Rückbildung geschädigter Zellen bzw. eine Isolation solcher Zellen oder ein einfrieren des bestehenden Niveaus. Eine antioxidative Wirkung ist nachweisbar und offensichtlich ist ein dermatologischer Schutz bzw. ein Schutz von Erbinformationen in betroffenen Körper- bzw. Hautzellen erreichbar.

## Patentansprüche

1. Mittel zur Verwendung zum Schutz von Hautzellen durch topische Applikation, welches als Inhaltsstoffe in wässeriger Lösung befindliche pflanzliche Wirkstoffe und Vitamine enthält, wobei ein Wirkstoff aus von Grüntee extrahierten Polyphenolen besteht und das Mittel weiterhin einen Anteil an Glutamat bzw. Glutaminsäure von 60 - 90 Gew.-%, bezogen auf den Gesamtgehalt an Aminosäuren, aufweist, und
wobei das Mittel durch die folgenden Verfahrensschritte hergestellt worden ist:
- Extrahieren pflanzlicher Wirkstoffe und Vitamine aus Grüntee-Blättern mittels Wasser bei Temperaturen im Bereich von 50 °C bis 120 °C und bei Drücken im Bereich von 1 x 10⁵ Pa bis 5 x 10⁵ Pa;
- Filtrieren des Extraktes mittels eines Filterbetts, ausgewählt aus der Gruppe, bestehend aus Oxidbett-Filter, Polymerbett-Filter und Mischformen davon; und
- Konzentrieren des Extraktes.

2. Mittel zur Verwendung nach Anspruch 1, wobei beim Konzentrieren des Extraktes eine Aufkonzentration der von Wasser verschiedenen Inhaltsstoffe auf 40 Gew.-% bis 100 Gew.-% erfolgt.

3. Mittel zur Verwendung nach Anspruch 1 oder 2, wobei die Vitamine ausgewählt sind aus der Gruppe, bestehend aus Vitamin A, Vitamin C, Vitamin E und Kombinationen davon.

4. Mittel zur Verwendung nach einem der vorangehenden Ansprüche, wobei das Mittel weiterhin Isoflavone enthält.

5. Mittel zur Verwendung nach einem der vorangehenden Ansprüche, wobei das Mittel keine organischen Lösungsmittel enthält.

6. Mittel zur Verwendung nach einem der vorhergehenden Ansprüche, wobei das Mittel mikro- oder nanoverkapselt ist.

7. Verwendung des Mittels nach einem der Ansprüche 1 bis 6 zur Herstellung eines Hautschutzprodukts zum Schutz vor Zellschäden bei Bestrahlung mit elektromagnetischen Strahlen durch topische Applikation.

8. Sonnenschutzmittel, umfassend das Mittel nach einem der Ansprüche 1 bis 6 und einen chemischen oder physikalischen UV-Filter, zur Verwendung zum Schutz vor UV-bedingten Hautschäden.

9. Sonnenschutzmittel zur Verwendung nach Anspruch 8, enthaltend 0,4 % des Mittels im Endprodukt.

## Claims

1. Preparation for use in the protection of skin cells by topical application, which comprises as ingredients vegetable active ingredients and vitamins contained in aqueous solution, wherein one active ingredient consists of polyphenols extracted from green tea, and the preparation further comprising a fraction of glutamate or glutamic acid of 60 - 90 percent by weight, relative to the total amount of amino acids, and wherein the preparation is produced by the following method steps:
- extracting vegetable active ingredients and vitamins from leaves of green tea using water at temperatures in the range of 50 °C to 120 °C and at pressures in the range of 1 x 10⁵ Pa to 5 x 10⁵ Pa;
- filtrating the extract using a filter bed selected from the group consisting of oxide bed filter, polymer bed filter and mixed forms thereof; and
- concentrating the extract.

2. Preparation for use according to claim 1, wherein during concentration of the extract a concentration of the ingredients other than water to 40 percent by weight up to 100 percent by weight is achieved.

3. Preparation for use according to claim 1 or 2, wherein the vitamins are selected from the group consisting of vitamin A, vitamin C, vitamin E and combinations thereof.

4. Preparation for use according to any one of the preceding claims, wherein the preparation further comprises isoflavones.

5. Preparation for use according to any one of the preceding claims, wherein the preparation does not comprise organic solvents.

6. Preparation for use according to any one of the preceding claims, wherein the preparation is microencapsulated or nanoencapsulated.

7. Use of the preparation according to any one of claims 1 to 6 for preparing a skin protection product for the protection of cell damages during radiation with electromagnetic radiation by topical application.

8. Sunscreen product comprising the preparation according to any one of claims 1 to 6 and a chemical or physical UV filter for use in the protection of UV related skin damages.

9. Sunscreen product for use according to claim 8 comprising 0.4 % of the preparation in the end product.

## Revendications

1. Produit destiné à l'utilisation pour la protection des cellules cutanées par application topique, qui en tant que composants contient des principes actifs végétaux et des vitamines se trouvant dans une solution aqueuse, un principe actif consistant dans des polyphénols extraits de thé vert et le produit présentant par ailleurs une part de 60 à 90 % en poids d'acides glutamiques en rapport à la teneur totale en acides aminés et le produit ayant été fabriqué par les étapes de procédé suivantes:
- Extraction de principes actifs végétaux et de vitamines de feuilles de thé vert au moyen d'eau à des températures de l'ordre de 50 °C à 120 °C et à des pressions de l'ordre de 1 x 10⁵ Pa à 5 x 10⁵ Pa ;
- Filtration de l'extrait au moyen d'une couche filtrante, choisie dans le groupe comprenant les filtres à couches d'oxydes, les filtres à couches de polymère et les formes mixtes de ces derniers ; et
- Concentration de l'extrait.

2. Produit destiné à l'utilisation selon la revendication 1, lors de la concentration de l'extrait, une concentration des composants différents de l'eau s'effectuant à de 40 % à 100 % en poids.

3. Produit destiné à l'utilisation selon la revendication 1 ou la revendication 2, les vitamines étant choisies dans le groupe composé de la vitamine A, de la vitamine C, de la vitamine E et d'associations de ces dernières.

4. Produit destiné à l'utilisation selon l'une quelconque des revendications précédentes, le produit contenant par ailleurs des isoflavones.

5. Produit destiné à l'utilisation selon l'une quelconque des revendications précédentes, le produit ne contenant aucun solvant organique.

6. Produit destiné à l'utilisation selon l'une quelconque des revendications précédentes, le produit étant micro-encapsulé ou nano-encapsulé.

7. Utilisation du produit selon l'une quelconque des revendications 1 à 6 pour la fabrication d'un produit de protection cutanée pour la protection contre l'endommagement cellulaire lors de l'irradiation aux rayons électromagnétiques par application topique.

8. Produit de protection solaire, comprenant le produit selon l'une quelconque des revendications 1 à 6 et un filtre UV chimique ou physique destiné à l'utilisation pour la protection des dommages de la peau dus aux UV.

9. Produit de protection solaire destiné à l'utilisation selon la revendication 8, contenant 0,4 % du produit dans le produit fini.
